# EUROPEAN PATENT APPLICATION

(11) **EP 3 777 674 A1**
(43) Date of publication of application: **17.02.2021**
(21) Application number: 19777828.5
(22) Date of filing: 27.03.2019
(51) Int. Cl.: A61B 5/0456, A61B 5/0464, A61B 5/00, A61B 5/04

(54) **TIME SERIES DATA LEARNING AND ANALYZING METHOD USING ARTIFICIAL INTELLIGENCE**

(30) Priority: 30.03.2018 KR 20180037077; 30.05.2018 KR 20180061857
(71) Applicant: Samsung SDS Co., Ltd., Seoul 05510 (KR)
(72) Inventor: OH, Kyu Sam, Seoul 05510 (KR); JEON, Eun Joo, Seoul 05510 (KR); KWON, Soon Hwan, Seoul 05510 (KR); SON, Hyeong Gwan, Seoul 05510 (KR); YUN, Yong Keun, Seoul 05510 (KR); KIM, Min Soo, Seoul 05510 (KR); YEO, Hyun Joo, Seoul 05510 (KR)
(74) Representative: Glawe, Delfs, Moll
(86) International application number: PCT/KR2019/003540
(87) International publication number: WO 2019/190185

(57) **Abstract**

Provided is a method for learning and analyzing time series data using artificial intelligence. According to an embodiment of the inventive concept, a time series data analysis method is performed by a computing device, the method comprises inputting, for each of a plurality of units, in which the time series data is split on a time axis, a feature of each of the units to an intermediate neural network, obtaining m-dimensional intermediate output data (m is a natural number of 2 or more) from the intermediate neural network, inputting the intermediate output data of a plurality of units immediately adjacent in time to a final neural network to obtain final output data output from the final neural network, and generating an analysis result of the time series data using the final output data.

## Description

### BACKGROUND

### 1. Field

The inventive concept relates to a method for learning and analyzing time series data using artificial intelligence. Specifically, the inventive concept relates to a method for performing learning and analysis of time series data using a plurality of neural networks.

### 2. Description of the Related Art

With the digitization of information and the development of data storage technology, a large amount of data is accumulated and machine learning technology is introduced and utilized in various fields. Machine learning is a technique that analyzes a large number of input data to stochastically classify objects or predict values within a specific range. Machine learning operates in the way of empirically analyzing a large number of input data and stochastically deriving a result rather than deriving a result by a specific rule.

However, in learning and analyzing time series data using artificial intelligence, there is a problem in that time series data may have a very long length of data to be processed in comparison with image data. Taking electrocardiogram data as an example, it is necessary to comprehensively learn and analyze electrocardiogram data measured for 24 hours or more to derive a diagnosis result, which is not only time-consuming during learning and analysis, but also causes the initial input data to be gradually blurred and diluted, thereby occurring problems related to accuracy when learning and analyzing long-term data.

### SUMMARY

An embodiment of the inventive concept is to provide a method and apparatus for reducing the computing amount of time series data learning and analysis using artificial intelligence.

Another embodiment of the inventive concept is to provide a method and apparatus that can improve the accuracy of time series data learning and analysis using artificial intelligence.

An embodiment of the inventive concept is to provide a method and apparatus for reducing the computing amount of electrocardiogram (ECG) learning and analysis using artificial intelligence.

An embodiment of the inventive concept is to provide a method and apparatus that can improve the accuracy of ECG learning and analysis using artificial intelligence.

Embodiments of the inventive concept are not limited to the above-mentioned embodiments, and other embodiments not mentioned will be clearly understood by those skilled in the art from the following description.

According to an embodiment of the inventive concept, a time series data analysis method is performed by a computing device, the method comprises inputting, for each of a plurality of units, in which the time series data is split on a time axis, a feature of each of the units to an intermediate neural network, obtaining m-dimensional intermediate output data (m is a natural number of 2 or more) from the intermediate neural network, inputting the intermediate output data of a plurality of units immediately adjacent in time to a final neural network to obtain final output data output from the final neural network, and generating an analysis result of the time series data using the final output data.

According to an embodiment of the inventive concept, wherein the intermediate neural network and the final neural network are RNN (Recurrent Neural Network).

According to an embodiment of the inventive concept, wherein nodes of an input layer of the final neural network are m.

According to an embodiment of the inventive concept, wherein obtaining the intermediate output data comprises, inputting, by a first computing device, a feature of a first unit included in the plurality of units to the intermediate neural network implemented in the first computing device and obtaining m-dimensional intermediate output data from the intermediate neural network; and inputting, by a second computing device, a feature of a second unit included in the plurality of units to an intermediate neural network implemented in the second computing device and obtaining m-dimensional intermediate output data from the intermediate neural network.

According to an embodiment of the inventive concept, wherein obtaining the final output data comprises, receiving, by a third computing device, intermediate output data of the first unit from the first computing device, and intermediate output data of the second unit from the second computing device, and sequentially inputting intermediate output data of the first unit and intermediate output data of the second unit to the final neural network, wherein the first unit and the second unit are immediately adjacent units in time.

According to an embodiment of the inventive concept, wherein the intermediate neural network comprises k levels (k is a natural number of 2 or more), wherein inputting to the intermediate neural network comprises, inputting a feature of each of the plurality of units to a level 1 intermediate neural network; inputting level i (i is a natural number of 1 or more) output data obtained from a level i intermediate neural network to a level i+1 intermediate neural network; and obtaining level i + 1 output data from a level i + 1 intermediate neural network, wherein the intermediate output data is data output from a level k intermediate neural network.

According to an embodiment of the inventive concept, wherein the time series data is electrocardiogram data.

According to an embodiment of the inventive concept, wherein the analysis result is an electrocardiogram diagnosis result.

According to an embodiment of the inventive concept, wherein the n units are beat units that are split using R-peak.

According to an embodiment of the inventive concept, wherein the split n beat units are obtained by splitting the time series data and comparing similarities between split beats to replace a beat having a similarity to a preceding specific beat equal to or more than a predetermined value with the feature unit.

According to an embodiment of the inventive concept, a time series data learning method is performed by a computing device, the method comprises inputting, for each of a plurality of units, in which the time series data is split on a time axis, a feature of each of the units to an intermediate neural network, obtaining m-dimensional (m is a natural number of 2 or more) intermediate output data from the intermediate neural network, inputting the intermediate output data of a plurality of units immediately adjacent in time to a final neural network to obtain final output data output from the final neural network, and comparing the final output data with a label attached to the time series data.

According to an embodiment of the inventive concept, wherein the intermediate neural network and the final neural network are RNN (Recurrent Neural Network).

According to an embodiment of the inventive concept, wherein the comparing step comprises, comparing the final output data with a label attached to the time series data to calculate an error, the method further comprises, back propagating the error to adjust a weight of the intermediate neural network and a weight of the final neural network after the comparing step.

According to an embodiment of the inventive concept, wherein adjusting the weight comprises, adjusting a weight of the intermediate neural network by making a learning rate of the intermediate neural network 1/n times a learning rate of the final neural network.

According to an embodiment of the inventive concept, wherein the intermediate neural network comprises k levels (k is a natural number of 2 or more), wherein inputting to the intermediate neural network comprises, inputting a feature of each of the plurality of units to a level 1 intermediate neural network; inputting level i (i is a natural number of 1 or more) output data obtained from a level i intermediate neural network to a level i+1 intermediate neural network; and obtaining level i+1 output data from a level i+1 intermediate neural network, wherein the intermediate output data is data output from a level k intermediate neural network.

According to an embodiment of the inventive concept, wherein the time series data is electrocardiogram data.

According to an embodiment of the inventive concept, wherein the n units are beat units that are split using a R-peak.

According to other embodiment of the inventive concept, a time series data analysis apparatus comprises a network interface for receiving time series data, one or more processors, a memory for loading a computer program performed by the processor, and a storage for storing the computer program, wherein the computer program comprises an instruction for inputting, for each of a plurality of units, in which the time series data is split on a time axis, a feature of each of the units to an intermediate neural network and obtaining m-dimensional (m is a natural number of 2 or more) intermediate output data from the intermediate neural network, an instruction for inputting the intermediate output data of a plurality of units immediately adjacent in time to a final neural network and obtaining final output data output from the final neural network, and an instruction for generating an analysis result of the time series data using the final output data.

According to another embodiment of the inventive concept, a time series data learning apparatus comprises a network interface for receiving time series data, one or more processors, a memory for loading a computer program performed by the processor, and a storage for storing the computer program, wherein the computer program comprises an instruction for inputting, for each of a plurality of units, in which the time series data is split on a time axis, a feature of each of the units to an intermediate neural network and obtaining m-dimensional (m is a natural number of 2 or more) intermediate output data from the intermediate neural network, an instruction for inputting the intermediate output data of a plurality of units immediately adjacent in time to a final neural network and obtaining final output data output from the final neural network, and an instruction for comparing the final output data with a label attached to the time series data.

According to another embodiment of the inventive concept, a computer readable recording medium stores a computer program which is configured to execute inputting, for each of a plurality of units, in which the time series data is split on a time axis, a feature of each of the units to an intermediate neural network, obtaining m-dimensional intermediate output data (m is a natural number of 2 or more) from the intermediate neural network, inputting the intermediate output data of a plurality of units immediately adjacent in time to a final neural network to obtain final output data output from the final neural network, and generating an analysis result of the time series data using the final output data.

According to various embodiments of the inventive concept described above, when performing time series data learning and analysis using artificial intelligence, the computing amount may be reduced and the accuracy may be improved.

According to various embodiments of the inventive concept described above, when performing electrocardiogram data learning and analysis using artificial intelligence, the computing amount may be reduced and the accuracy may be improved.

Effects of the inventive concept are not limited to the above-mentioned effects, and other effects not mentioned will be clearly understood by those skilled in the art from the following description.

### BRIEF DESCRIPTION OF THE DRAWINGS

FIGS. 1a and 1b are diagrams illustrating a wearable electrocardiogram measuring device and an attachment method thereof.
FIG. 2 is a diagram illustrating an electrocardiogram analysis system including an electrocardiogram measuring device, a network 210 and a server.
FIGS. 3a and 3b are diagrams illustrating a conventional RNN-based electrocardiogram analysis method.
FIG. 4 is a flowchart illustrating an electrocardiogram analysis method using artificial intelligence according to an embodiment of the inventive concept.
FIG. 5 is a diagram illustrating an electrocardiogram signal splitting method according to an embodiment of the inventive concept.
FIGS. 6a to 6c are diagrams illustrating a pseudo beat processing method according to an embodiment of the inventive concept.
FIGS. 7a and 7b are diagrams illustrating an intermediate neural network and a final neural network according to an embodiment of the inventive concept.
FIGS. 8 to 10 are diagrams illustrating a time series data analysis method using artificial intelligence according to an embodiment of the inventive concept.
FIG. 11 is a flowchart illustrating a time series data analysis method using artificial intelligence according to an embodiment of the inventive concept.
FIG. 12 is a diagram illustrating a case, where an intermediate neural network is implemented with a plurality of levels in a neural network structure according to an embodiment of the inventive concept.
FIG. 13 is a flowchart illustrating a time series data analysis method when an intermediate neural network is implemented with a plurality of levels in a neural network structure according to an embodiment of the inventive concept.
FIG. 14 is a flowchart illustrating an electrocardiogram learning method using artificial intelligence according to an embodiment of the inventive concept.
FIG. 15 is a diagram illustrating learning data used in an electrocardiogram learning method using artificial intelligence according to an embodiment of the inventive concept.
FIG. 16 is a diagram illustrating a time series data learning method using artificial intelligence according to an embodiment of the inventive concept.
FIG. 17 is a flowchart illustrating a time series data learning method using artificial intelligence according to an embodiment of the inventive concept.
FIG. 18 is a hardware configuration diagram of an exemplary computing device that can implement a time series data analysis method or time series data learning method using artificial intelligence according to embodiments of the inventive concept.

### DETAILED DESCRIPTION

Hereinafter, preferred embodiments of the inventive concept will be described with reference to the attached drawings. Advantages and features of the inventive concept and methods of accomplishing the same may be understood more readily by reference to the following detailed description of preferred embodiments and the accompanying drawings. The inventive concept may, however, be embodied in many different forms and should not be construed as being limited to the embodiments set forth herein. Rather, these embodiments are provided so that this disclosure will be thorough and complete and will fully convey the concept of the invention to those skilled in the art, and the inventive concept will only be defined by the appended claims. Like numbers refer to like elements throughout.

Unless otherwise defined, all terms including technical and scientific terms used herein have the same meaning as commonly understood by one of ordinary skill in the art to which this invention belongs. Further, it will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein. The terms used herein are for the purpose of describing particular embodiments only and is not intended to be limiting. As used herein, the singular forms are intended to include the plural forms as well, unless the context clearly indicates otherwise.

The terms "comprise", "include", "have", etc. when used in this specification, specify the presence of stated components, steps, operations, and/or elements but do not preclude the presence or addition of one or more other components, steps, operations, and/or elements.

Prior to the description of the specification, some terms used in the specification will be clarified.

In the technical field, to which the inventive concept belongs, the term 'neural network' refers to a graph structure composed of a plurality of layers, which are modeled after the neural network of living organisms, in particular, the human visual/auditory cortex, and a plurality of nodes constituting each layer. The neural network comprises one input layer, one or more hidden layers and one output layer.

In the technical field, to which the inventive concept belongs, the term 'input layer' refers to a layer that receives data to be analyzed/learned in a layer structure of a neural network.

In the technical field, to which the inventive concept belongs, the term 'output layer' refers to a layer, in which a result value is output in a layer structure of a neural network.

In the technical field, to which the inventive concept belongs, the term 'hidden layer' refers to all layers except the input layer and the output layer in the layer structure of the neural network. The neural network consists of a continuous layer of neurons, with neurons in each layer connected to neurons in the next layer. If the input layer is directly connected to the output layer without the hidden layer, each input contributes to the output independently of the other inputs, making it difficult to obtain accurate results. In practice, since input data is interdependent and coupled to each other to affect the output in a complex structure, subtle interactions between inputs where the neurons in the hidden layer affect the final output can be captured by adding the hidden layer. In other words, the hidden layer can be thought of as processing a high level features or attributes in the data.

In the technical field, to which the inventive concept belongs, the term 'neuron' is a concept, in which a neuron in biological neural networks corresponds to a neural network. The term 'neuron' is also referred to as 'node.'

In the technical field, to which the inventive concept belongs, the term 'weight' refers to a concept, in which a degree of synaptic connection is strengthened through repeated signal transmission in a biological neural network corresponds to a neural network. Frequent signal transmission from neuron 1 to neuron 2 in biological neural networks enhances the pathway from neuron 1 to neuron 2, i.e., the synaptic connection, for efficient signal transmission. This is regarded as a kind of learning or memory process, and the neural network describes this as the concept of 'weight.'

In the technical field, to which the inventive concept belongs, the term 'back propagation' is a word derived from 'backward propagation of errors,' and means adjusting the weight of the neural network through back propagation of an error that is a difference between an output value and an actual value.

In the technical field, to which the inventive concept belongs, the term 'gradient descent' is a method of determining the weight of a learning model in back propagation, and means a method of finding a weight, which minimizes a difference between an output value of a feedforward function value including the weight and a labeled actual value. For example, when the shape of the loss function is expressed as a parabolic shape, it is possible to calculate the weight by finding the lowest point, and since the process of finding the lowest point looks like descending a gradient hill, it is named the gradient descent.

In the technical field, to which the inventive concept belongs, the term 'learning rate' means the size of a step of finding the lowest value in the gradient descent algorithm for adjusting the weight of a neural network. Larger learning rates result in faster adjustments but less precision, and smaller learning rates provide higher precision but slower adjustments.

In the technical field, to which the inventive concept belongs, the term 'Convolutional Neural Networks (CNN)' is a deep learning model that combines a neural network with a filter technology, and is optimized such that the neural network can acquire features of input data better. Convolutional Neural Networks have shown excellent performance in the field of computer vision.

In the technical field, to which the inventive concept belongs, the term 'Recurrent Neural Network (RNN)' is a deep learning model suitable for learning data that changes over time, such as time series data. It is a neural network constituted by connecting networks at the reference time point (t) and the next time point (t+1). The Recurrent Neural Network has a feature that a connection between nodes has a recurrent structure, and this structure enables the neural network to effectively process time-varying features. The Recurrent Neural Network can effectively process input in the form of a sequence and has shown good performance in processing time series data having time-varying features, such as handwriting recognition and speech recognition.

Artificial intelligence related technologies and terms described above are described in detail on websites, such as Wikipedia and YouTube, and thus detailed descriptions thereof will be omitted. For example, The Recurrent Neural Network is described in detail in https://en.wikipedia.org/wiki/Recurrent_neural_network, and the like.

In the present specification, the term 'time series data' means data listed in chronological order. However, it is not necessary to be continuous in time, and even discrete data may be 'time series data' as used herein if they are listed in chronological order.

In the present specification, the term 'unit' means each entity when time series data is split into two or more entities on a time axis.

In the present specification, the term 'feature' means a feature of time series data or a unit extracted from the time series data or the unit.

In the present specification, the term 'intermediate neural network' means a neural network of an intermediate step that receives input data and outputs intermediate output data. The intermediate output data differs from an ordinary output value of a neural network in that the intermediate output data is not the value desired to be finally obtained using artificial intelligence.

In the present specification, the 'final neural network' refers to a neural network of a final step that receives intermediate output data from an intermediate neural network and outputs final output data. The final neural network is a neural network located at a terminal of an artificial intelligence system composed of a plurality of neural networks according to an embodiment of the inventive concept, and the final output data output from the final neural network is the value desired to be finally obtained using artificial intelligence.

The inventive concept to be described below relates to a time series data learning and analysis method using artificial intelligence. Specifically, the inventive concept discloses a time series data learning and analysis method that performs learning and analysis of time series data using a plurality of neural networks.

An electrocardiogram is illustrated as an example of time series data in order to specifically describe the inventive concept in the specification. Specifically, a time series data learning and analysis method using artificial intelligence according to an embodiment of the inventive concept will be described by explaining a process of using a plurality of neural networks in electrocardiogram learning and analysis according to an embodiment of the inventive concept.

Hereinafter, the inventive concept will be described in detail with reference to the accompanying drawings.

The electrocardiogram is a record of analysis of the heart's electrical activity in the form of wavelengths. The electrocardiogram is a record of the potential associated with the heartbeat at the body surface with shapes and is the most frequently used test for diagnosing circulatory diseases.

The electrocardiogram is frequently used to diagnose arrhythmias and coronary artery disease. If the arrhythmia occurs intermittently, it is necessary to obtain the electrocardiogram recorded during daily life because the arrhythmias cannot be diagnosed by only one electrocardiogram test. Infrequently occurring arrhythmias may not be detected by short-term electrocardiogram recordings, so if arrhythmias is suspected, the electrocardiogram is measured and recorded for a long time to make an accurate diagnosis.

The conventional electrocardiogram measuring device was a method of measuring by attaching a large number of electrodes to the body. For example, the Holter monitor, which is commonly used, is a method of measuring electrocardiogram by attaching 5 to 7 electrodes to the chest and wearing an inspection apparatus having a weight of about 500g on the waist. Since the conventional electrocardiogram measuring device is large and heavy in size and has a large number of electrodes, there is a problem that there is a sense of rejection when it is attached to a body and interferes with free daily life.

In order to solve this problem, a wearable typed electrocardiogram measuring device that has been recently developed reduces the size and weight of the device and minimizes the number of electrodes. The reason for minimizing the number of electrodes is to consider both the convenience of the user's daily life and the battery performance of the electrocardiogram measuring device.

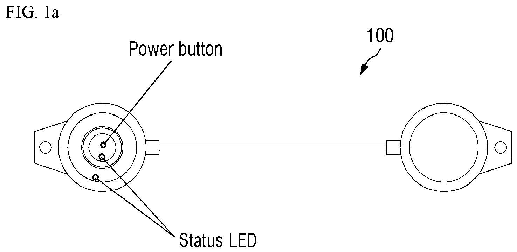

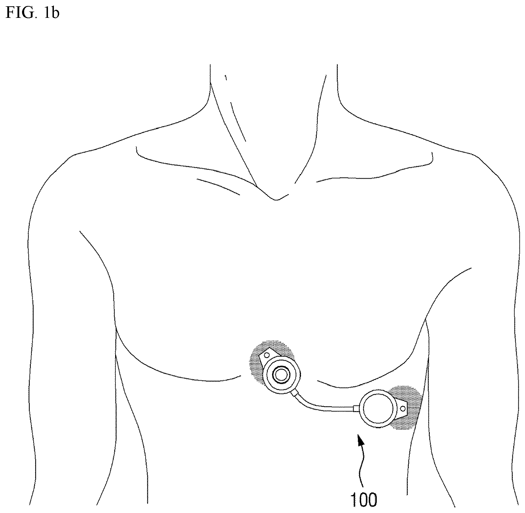

FIGS. 1a and 1b are diagrams illustrating a wearable electrocardiogram measuring device and an attachment method thereof.

FIG. 1a illustrates the wearable electrocardiogram measuring device 100, and as shown in FIG. 1a, the number of electrodes is two.

FIG. 1b illustrates a method of measuring an electrocardiogram by attaching the wearable electrocardiogram measuring device 100 to a body. As shown in FIG. 1b, an electrocardiogram is measured by attaching an electrode near the heart.

The wearable electrocardiogram measuring device 100 is easier to carry than the conventional electrocardiogram measuring device. However, while the conventional electrocardiogram measuring device analyzes the electrocardiogram signal of a multi-channel by using a plurality of electrodes and enables the relatively accurate measurement and diagnosis of the electrocardiogram, the wearable electrocardiogram measuring device 100 has a problem that only the electrocardiogram signal of one channel is measured and thus the accuracy of electrocardiogram measurement and diagnosis is inferior.

In the wearable electrocardiogram measuring device 100, there is a difficulty that an electrocardiogram analysis method used in the conventional electrocardiogram measuring device collecting multi channels, such as a Holter ECG measuring device, cannot be used as it is, and arrhythmias should be diagnosed using only one channel of the electrocardiogram signal. Nevertheless, since the wearable electrocardiogram measuring device 100 has strengths, such as convenience of carrying, many researches are going on a method of diagnosing arrhythmias using only the electrocardiogram signal of one channel measured by the wearable electrocardiogram measuring device 100. In particular, due to the development of deep learning techniques in artificial intelligence field, there might be a possibility to achieve arrhythmia diagnosis of sufficiently high accuracy with only one signal.

However, a device for measuring the electrocardiogram of the subject in the inventive concept is not limited to the wearable typed electrocardiogram measuring device 100, but any type of electrocardiogram measuring device 100 may be provided as long as it can be attached to the body and measure the electrocardiogram. Hereinafter, for the convenience of description, a device for measuring an electrocardiogram of a subject in the inventive concept is unified as an 'electrocardiogram measuring device100.'

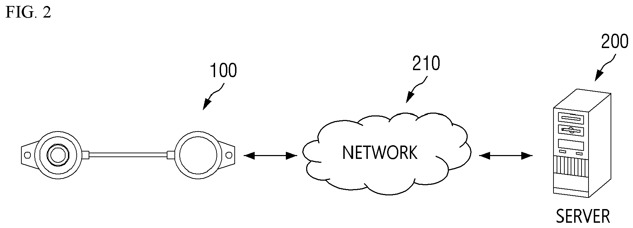

FIG. 2 is a diagram illustrating an electrocardiogram analysis system including an electrocardiogram measuring device, a network and a server.

Since the electrocardiogram measuring device 100 only measures the electrocardiogram of a subject, and in many cases does not provide an analysis function by itself, it is preferred that the electrocardiogram measuring device 100 only performs electrocardiogram measurement and the electrocardiogram analysis is performed at the server 200 stage. Specifically, it is preferred that the electrocardiogram measuring device 100 measures an electrocardiogram and transmits the electrocardiogram to the server 200, and the server 200 stage analyzes the electrocardiogram data using artificial intelligence and the like.

As shown in FIG. 2, an electrocardiogram analysis system according to an embodiment of the inventive concept may include an electrocardiogram measuring device 100, a network 210 and a server 200.

The subject lives daily life with the electrocardiogram measuring device 100 attached thereto. The electrocardiogram data measured by the electrocardiogram measuring device 100 is transmitted to the server 200 through the network 210. In this case, the server 200 may be a server of a hospital, a server of a medical service provider, a server of an IT company or a server of a government agency such as the Ministry of Health and Welfare. Since the electrocardiogram data of the individual is sensitive medical information, the server 200 is preferably a server of a company that is allowed to collect and analyze medical information.

The server 200 performs learning and analysis using the electrocardiogram data received from the electrocardiogram measuring device 100. When the server 200 learns the electrocardiogram data, a medical professional, such as a cardiologist, may allow the electrocardiogram data labeled with the electrocardiogram diagnosis result to be learned.

Due to the nature of artificial intelligence, the learning of the neural network requires a long time and high computing power, while the relatively short time and low computing power are sufficient for the application of the neural network. Therefore, the learning of the electrocardiogram data is to be performed at the server 200 stage, but the analysis of the electrocardiogram data may be performed in the electrocardiogram measuring device 100 itself, a separate electrocardiogram analysis device connected to the electrocardiogram measuring device 100 via wired/wireless, and a smartphone, a tablet, a notebook computer and the like connected to the electrocardiogram measuring device 100 via wired/wireless.

However, in the present specification, an electrocardiogram data learning and analysis method according to an embodiment of the inventive concept will be described on the premise that both learning and analysis of the electrocardiogram data are performed in the server 200. This description does not exclude that the learning and analysis of electrocardiogram data are performed in the electrocardiogram measuring device 100 itself, a separate electrocardiogram analysis device connected to the electrocardiogram measuring device 100 via wired/wireless, and a smartphone, a tablet, a notebook computer and the like connected to the electrocardiogram measuring device 100 via wired/wireless.

The learning and analysis method of electrocardiogram data using artificial intelligence, which has been researched so far, is based on CNN. The CNN is a deep learning model that combines neural networks with filter technology, and has shown excellent performance in the computer vision field, such as image analysis. However, since the electrocardiogram is time series data having a sequential context, unlike an image, there is a limit in analyzing the electrocardiogram using a CNN technique specialized for image analysis.

The CNN-based electrocardiogram analysis method does not read the sequential context of the entire electrocardiogram signal, but rather identifies the features of a part of the electrocardiogram signal and combines these features to derive the analysis result of the entire electrocardiogram. Since the CNN-based electrocardiogram analysis method analyzes electrocardiogram data from the microscopic view rather than the macroscopic view, when a part of the electrocardiogram signal contains noise, it is difficult to grasp the feature of the corresponding part, thereby adversely affecting the entire electrocardiogram signal discrimination accuracy. Further, when there are a number of conflicting features in one signal, the filtered feature is not considered in the diagnosis, which leads to a problem that the discrimination accuracy is significantly reduced.

In order to overcome this problem, research on the electrocardiogram analysis method using RNN is in progress.

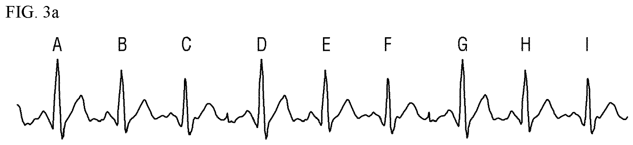

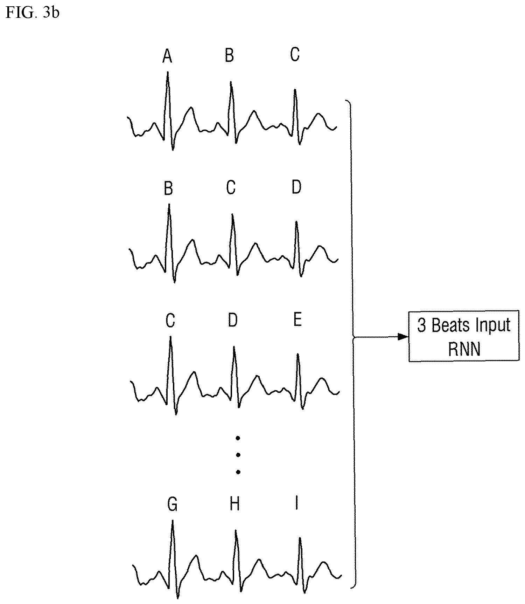

FIGS. 3a and 3b are diagrams illustrating a conventional RNN-based electrocardiogram analysis method.

FIG. 3a illustrates an electrocardiogram signal measured for a predetermined time in the electrocardiogram measuring device 100.

The sharply raised portion of the electrocardiogram signal illustrated in FIG. 3a is R-peak. R-peak is described in detail in https://en.wikipedia.org/wiki/QRS_complex and the like, so the detailed description is omitted.

FIG. 3b illustrates that an electrocardiogram signal measured for a predetermined time in the electrocardiogram measuring device 100 is input to the RNN in the unit of three beats (which refers to the heartbeat unit).

The electrocardiogram signal measured for a predetermined time is split in the beat unit using R-peak. Three immediately adjacent beats in time are input to the RNN together, because the relationship between the previous beat, the beat to be discriminated and the next beat is important for discriminating arrhythmia from the electrocardiogram signal. In other words, this is because that the occurrence interval and the height difference between the previous beat, the beat to be discriminated and the next beat are important consideration factors for arrhythmia discrimination.

When an electrocardiogram signal is divided into 9 beats and each beat is named A, B, C, D, E, F, G, H and I, A+B+C, B+C+D, C+D+E, D+E+F, E+F+G, F+G+H and G+H+I are input to the RNN

As shown in FIG. 3b, A+B+C, B+C+D, C+D+E, D+E+F, E+F+G, F+G+H and G+H+I are input to one RNN sequentially.

When one beat is sampled at 250 Hz and the electrocardiogram signal of FIG. 3a is analyzed using a conventional RNN, 7 (number of beats to be discriminated) x 3 (three adjacent beats are input together) x 250 (sampling number of each beat) = 5250 length input should be processed.

In addition, since arrhythmia does not always appear, it is necessary to collect and analyze an electrocardiogram signal of the subject over a long period of time. Healthy adults have a heartbeat of 60 to 100 beats per minute, which translates into 86,400 to 144,000 beats per 24 hours. Assuming that a heartbeat is 80 beats per minute, a 24-hour heartbeat is 115,200 beats. This means that 80 beats are measured in 1 minute and 115,200 beats are measured in 24 hours.

To discriminate each of the 115,200 beats, the previous beat and next bit for each beat should be considered together, so when sampling one beat at 250 Hz, approximately 115,200 (the number of beats to be discriminated) × 3 (adjacent three beats are input together) x 250 (sampling number of each beat) = 86,400,000 length input should be processed by the RNN

The CNN-based electrocardiogram analysis is faster than the RNN-based electrocardiogram analysis, but the accuracy of analysis is inferior because it cannot read the sequential context of electrocardiogram signals. On the other hand, the RNN-based electrocardiogram analysis can read the sequential context of electrocardiogram signals, but it is difficult to guarantee real-time electrocardiogram analysis because the length of the input to be processed is too long.

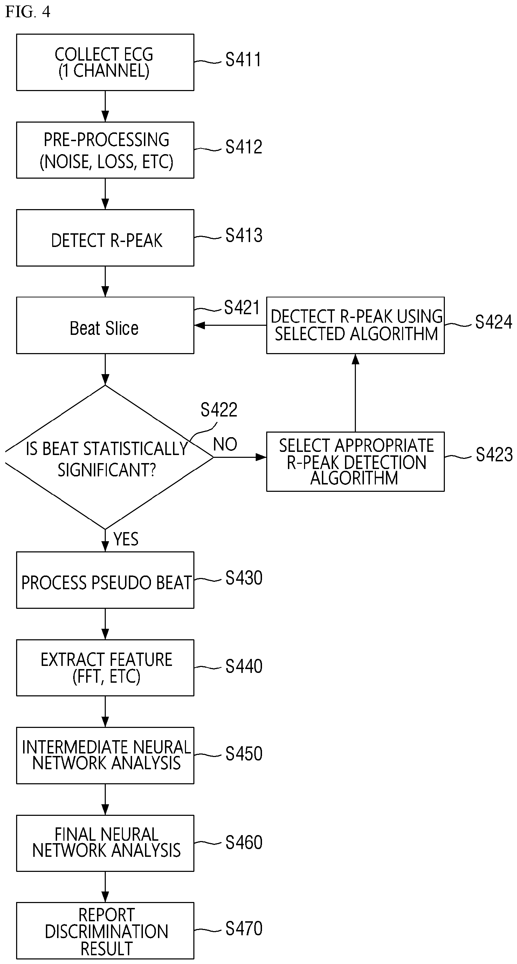

FIG. 4 is a flowchart illustrating an electrocardiogram analysis method using artificial intelligence according to an embodiment of the inventive concept.

As shown in FIG. 4, the electrocardiogram analysis method using artificial intelligence according to an embodiment of the inventive concept may comprise the electrocardiogram signal collecting step (S411), the electrocardiogram signal preprocessing step (S412), the R-peak detecting step (S413), the electrocardiogram signal splitting step (S421), the beat significance determining step (S422), the pseudo beat processing step (S430), the feature extracting step (S440), the intermediate neural network analysis step (S450), the final neural network analysis step (S460) and the discrimination result reporting step (S470).

The electrocardiogram signal collecting step (S411) is a step, in which the server 200 receives and collects an electrocardiogram signal from the electrocardiogram measuring device 100.

The electrocardiogram signal preprocessing step (S412) is a step of processing noise, signal disconnection, signal interference and the like mixed in the electrocardiogram signal. Specifically, the electrocardiogram signal received from the electrocardiogram measuring device 100 may be preprocessed using a conventionally known method such as a Band Pass Filter.

The R-peak detecting step (S413) is a step of detecting a R-peak in the electrocardiogram signal. When detecting the R-peak, it is not limited to a specific detection algorithm, but various conventionally known detection algorithms, such as Pan-Tompkins algorithm, Hilbert transform, Kathirvel et al (An efficient R-peak detection based on new nonlinear transformation and first-order Gaussian differentia-tor. Cardiovascular Engineering and Technology) can be used.

The electrocardiogram signal splitting step S421 is a step of splitting the electrocardiogram signal by beat unit using the detected R-peak.

The beat significance determining step (S422) is a step of determining whether the beat split using the R-peak corresponds to the actual heartbeat. For example, if sudden noise is incorrectly determined as the R-peak, the beat split based on the incorrectly determined R-peak does not correspond to the actual heartbeat.

Since human heartbeat tends to maintain similar lengths, a too short or long beat can be considered as false detection during the R-peak detecting step. Specifically, if a beat split using the R-peak is statistically too long (e.g., equal to or greater than 99 percentile) or too short (e.g., equal to or less than 1 percentile), it can be considered that false detection occurs in the R-peak detecting step. In this case, the R-peak should be detected again. The algorithm used to detect the R-peak again can be an algorithm other than the previously used algorithm or the previously used algorithm with modified parameters. Different algorithms may be applied to a statistically too long beat or too short beat respectively. The reason for detecting the R-peak again is that the R-peak detecting algorithm does not guarantee 100% accuracy, especially a wearable device may generate a lot of noise during data measurement and transmission.

If the beat split in the beat significance determining step (S422) is not statistically significant ('NO' of S422), another suitable R-peak detection algorithm is selected (S423), and the selected R-peak detection algorithm is used to detect the R-peak again (S424). The electrocardiogram signal is split by the beat unit again using the R-peak detected by using the selected R-peak detection algorithm. The process of selecting an R-peak detection algorithm and detecting the R-peak is repeated until the split beat is determined to be statistically significant.

In the beat significance determining step (S422), if the split beat is statistically significant ('YES' of S422), the pseudo beat processing step (S430) is performed. The pseudo beat processing step (S430) is a step of replacing a current beat with a previously generated beat if there is a beat similar to the current bit among the previously generated beats. The pseudo beat processing step (S430) is not essential, but the computing amount of a computer may be reduced through the pseudo beat processing step (S430).

The feature extracting step (S440) is a step of extracting a feature of each beat. The feature extraction can use a variety of methods widely used in signal processing, such as Fast Fourier Transform (FFT), Mel Frequency Cepstral Coefficient (MFCC), Filter Bank and Wavelet.

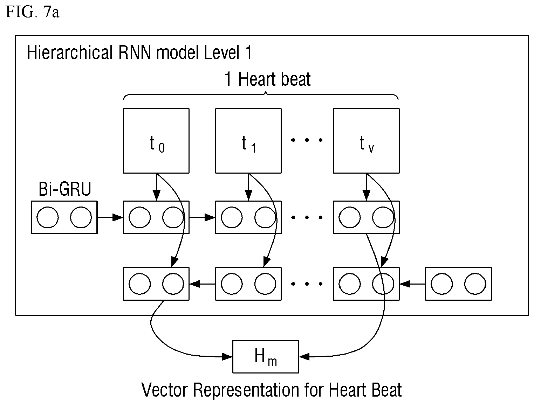

The intermediate neural network analysis step (S450) is a step of obtaining the intermediate output data by inputting the feature of each beat to the intermediate neural network. The intermediate output data differs from the ordinary output values of neural networks in that the intermediate output data is not the value desired to be finally obtained using artificial intelligence. Detailed descriptions of the intermediate neural network and the intermediate neural network analysis step (S450) will be described in detail later with reference to FIGS. 7a to 12.

The final neural network analysis step (S460) is a step of obtaining final output data by receiving intermediate output data from the intermediate neural network. The final neural network is a neural network located at a terminal of an artificial intelligence system composed of a plurality of neural networks according to an embodiment of the inventive concept, and the final output data output from the final neural network is the value desired to be finally obtained by using artificial intelligence. Detailed descriptions of the final neural network and the final neural network analysis step (S460) will be described in detail later with reference to FIGS. 7a to 12.

The discrimination result reporting step (S470) is a step of generating and reporting a diagnosis result using the final output data obtained in the final neural network analysis step (S460). Specifically, the discrimination result reporting step (S470) may be to analyze the electrocardiogram signal to discriminate whether the subject has arrhythmia and then report the discrimination result.

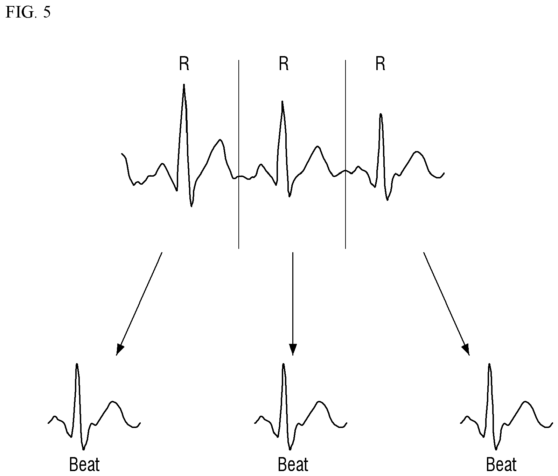

FIG. 5 is a diagram illustrating an electrocardiogram signal splitting method according to an embodiment of the inventive concept.

As shown in FIG. 5, the electrocardiogram signal may be split by the unit of beat based on the R-peak. In detail, the left side of the R-peak or the right side of the R-peak may be split in the electrocardiogram signal. If the electrocardiogram signal is split based on the R-peak, the position for splitting the electrocardiogram signal may be any position. In general, the waveform of the heartbeat is divided into P-QRS-T, and FIG. 5 reflects this to split the electrocardiogram signal so that the R-peak is at the center of each beat.

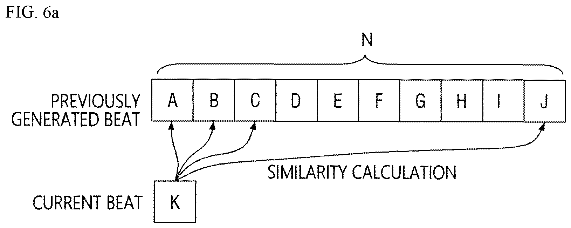

FIGS. 6a to 6c illustrate a pseudo beat processing method according to an embodiment of the inventive concept.

Long-term data collection is required to discriminate arrhythmias using electrocardiogram signals. Since arrhythmia is characterized by an irregular occurrence, it is not possible to determine the time of occurrence. Therefore, the electrocardiogram measuring device 100 in the form of a wearable device is attached to a subject to measure the electrocardiogram for a long time over several days.

When the server 200 analyzes the electrocardiogram signal by transmitting the electrocardiogram signal measured by the electrocardiogram measuring device 100 to the server 200, if there is a similar beat among the beats that have already generated before inputting data to the neural network, the analysis speed can be faster by analyzing only the previously generated beats and using the results together.

As shown in FIG. 6a, the similarity is calculated by comparing the current beat K with the previously generated beats A, B, C, D, E, F, G, H, I and J. The similarity between the beat to be currently determined and the N beats previously generated is calculated, and the most similar beat among the previously generated beats similar to or above a threshold is selected as the replacement beat.

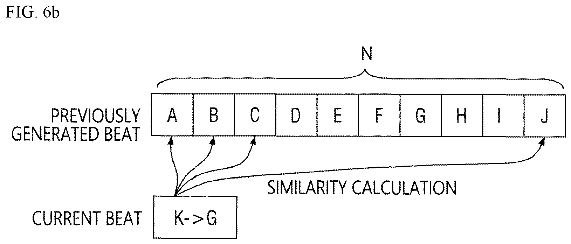

If the most similar beat among the previously generated beats similar to the current beat K equal to or above a threshold is beat G (see FIG. 6b), beat G is inserted instead of beat K. If there is no similar beat, beat K is maintained.

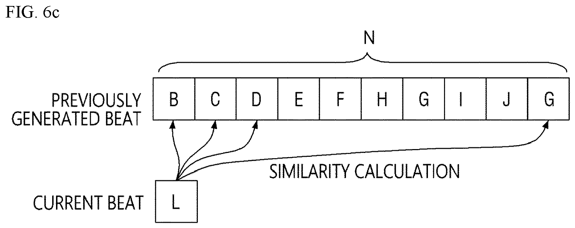

Since only N previous beat data are maintained, when the similarity of the next beat is determined, the situation is as shown in FIG. 6c.

By using the similarity-based beat connection information generated in this way, the analysis result of the beat G is shared without performing a separate analysis on the beat K. Further, the connection information generated in this step is used to restore the result of beat K after the diagnosis is complete. The similarity calculation algorithm may use any of the well-known similarity algorithms, such as L1, L2, and DTW. Since human electrocardiogram tends to repeat similar signals, the computing amount can be greatly reduced through such similarity-based beat filtering.

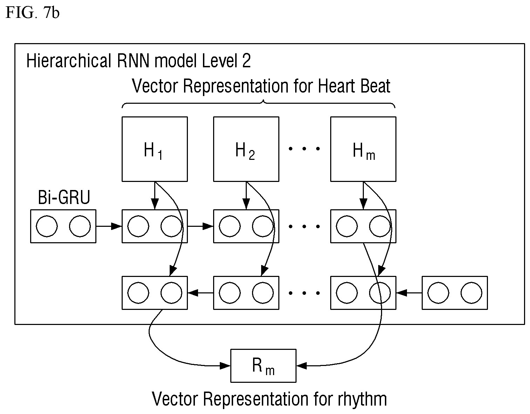

FIGS. 7a and 7b are diagrams illustrating an intermediate neural network and a final neural network according to an embodiment of the inventive concept.

According to the electrocardiogram analysis method according to an embodiment of the inventive concept as described above, after splitting the electrocardiogram signal by the unit of beat using the R-peak, the features of each beat are extracted and the features of each beat are input to the intermediate neural network.

FIG. 7a is a diagram illustrating an intermediate neural network in accordance with an embodiment of the inventive concept. As shown in FIG. 7a, when features t0, t1, ..., tv are extracted for any one beat, e.g., beat A, the v+1 features of beat A are input to v+1 nodes in the input layer of the intermediate neural network.

Data input to the intermediate neural network is output to m nodes in the output layer via the hidden layer. The value output to the m nodes in the output layer of the intermediate neural network may indicate the feature of beat A. It should be noted, however, that the value output to the m nodes in the output layer of the intermediate neural network is not the value desired to be finally obtained by comprehensively analyzing the electrocardiogram signal using artificial intelligence, but rather the value, in which the feature of any one beat, for example beat A, is expressed in the m dimension. The intermediate neural network may be one learning beat A through a map model, and in this case, the value output to m nodes in the output layer of the intermediate neural network may be any prediction value or classification value for beat A. The intermediate neural network may be one learning beat A through an unsupervised model, and in this case, the value output to m nodes in the output layer of the intermediate neural network may be a value for clustering beat A. In any case, the value output to the m nodes in the output layer of the intermediate neural network is only that the features of beat A are expressed in the m dimension, but is not the value desired to be finally obtained using artificial intelligence. It is preferable that two or more features are extracted at this time. That is, it is preferable that m is a natural number of 2 or more.

Meanwhile, although FIG. 7A illustrates that the intermediate neural network has an RNN structure, the intermediate neural network of FIG. 7a is not limited to the RNN structure, and may have a CNN structure or other neural network structures.

FIG. 7b is a diagram illustrating a final neural network according to an embodiment of the inventive concept. As shown in FIG. 7b, values output from m nodes of the output layer of the intermediate neural network are input to m nodes in the input layer of the final neural network.

The final neural network may be the RNN, in which case, in order to reflect the time-varying features of consecutive beats, the m-dimensional feature value for the previous beat of the beat to be discriminated, the m-dimensional feature value for the beat to be discriminated and the m-dimensional feature value for the next beat of the beat to be discriminated may be sequentially input to the input layer of the final neural network.

The electrocardiogram signal is divided into nine beats as shown in FIGS. 3a and 3Bb and each beat is named A, B, C, D, E, F, G, H, and I. When the beat to be discriminated, the previous beat and the next beat are input together and analyzed, the m-dimensional feature value for beat A, the m-dimensional feature value for beat B and the m-dimensional feature value for beat C may be input sequentially to the final neural network. When the entire electrocardiogram signal is divided into 9 beats, and each beat is A, B, C, D, E, F, G, H and I, data may be input to the final neural network in the following order.
(1) The m-dimensional feature value for beat A, the m-dimensional feature value for beat B and the m-dimensional feature value for beat C.
(2) The m-dimensional feature value for beat B, the m-dimensional feature value for beat C and the m-dimensional feature value for beat D.
(3) The m-dimensional feature value for beat C, the m-dimensional feature value for beat D and the m-dimensional feature value for beat E.
(4) The m-dimensional feature value for beat D, the m-dimensional feature value for beat E and the m-dimensional feature value for beat F.
(5) The m-dimensional feature value for beat E, the m-dimensional feature value for beat F and the m-dimensional feature value for beat G.
(6) The m-dimensional feature value for beat F, the m-dimensional feature value for beat G and the m-dimensional feature value for beat H.
(7) The m-dimensional feature value for beat G, the m-dimensional feature value for beat H and the m-dimensional feature value for beat I.

Data input to the final neural network is output to c nodes in the output layer via the hidden layer.

The final neural network may be one learning the entire electrocardiogram signal through a map model. In this case, the value output to the c nodes in the output layer of the final neural network may be a value for classifying the entire electrocardiogram signal into c classifications.

As shown in FIG. 7b, the final neural network has preferably an RNN structure that reflects time-varying features of data well. The RNN structure has a feature, in which the connection between nodes has a recursive structure, and thus it can effectively process time-varying features. Since the final neural network is for receiving m-dimensional features of consecutive beats sequentially and classifying the entire electrocardiogram signal, which is a set of consecutive beats, into c classifications, the structure of the final neural network is preferably an RNN structure. However, the final neural network is not limited to the RNN structure and may be any other structure as long as it is a neural network structure that can reflect the time-varying feature of time series data. That is, the final neural network is not limited to the RNN structure.

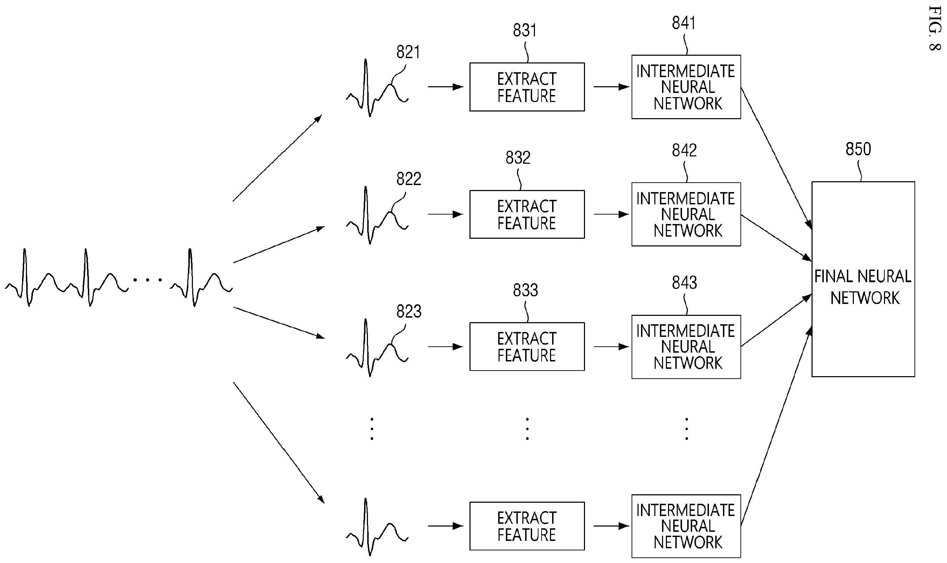

FIGS. 8 to 10 are diagrams illustrating a time series data analysis method using artificial intelligence according to an embodiment of the inventive concept.

FIG. 8 illustrates an example of analyzing an electrocardiogram signal using a time series data analysis method using artificial intelligence according to an embodiment of the inventive concept. As described above, this is only for the convenience of description, and the time series data analysis method using artificial intelligence according to an embodiment of the inventive concept is not limited to electrocardiogram signal analysis.

Referring to FIG. 8, a process of analyzing time series data, for example, an electrocardiogram signal, using artificial intelligence according to an embodiment of the inventive concept will be described.

When the electrocardiogram signal 810 is input, the electrocardiogram signal is split into a plurality of beats 821, 822, 823 ... according to a predetermined criterion. For example, after detecting the R-peak from the electrocardiogram signal 810, the electrocardiogram signal may be split into a plurality of beats 821, 822, 823 ... using the R-peak.

When generalizing this to time series data, when time series data is input, it may be described as splitting the time series data into a plurality of units according to a predetermined criterion.

Next, for each of the plurality of beats 821, 822, 823..., an operation of extracting the features 831, 832, 833 ... of each beat is performed. Feature extraction can use a variety of methods widely used in signal processing, such as Fast Fourier Transform (FFT), Mel Frequency Cepstral Coefficient (MFCC), Filter Bank and Wavelet.

When generalizing this to time series data, when time series data is split into a plurality of units, it may be described as extracting a feature of each unit for each of the plurality of units.

Next, an operation of inputting the features 831, 832, 833 ... of each beat into the intermediate neural networks 841, 842, 843... is performed. If the electrocardiogram signal is split into n beats, all n features would be extracted. In this case, the intermediate neural networks are also preferably n. A feature of each of the n beats is input to each input layer of the n intermediate neural networks to obtain m-dimensional feature values for each of the n beats in the output layer of each of the n intermediate neural networks. In this case, the value output from the intermediate neural network is referred to as 'intermediate output data.'

Although an operation of extracting a plurality of features 831, 832, 833 ... from the plurality of beats 821, 822, 823 ... is performed by a well-known algorithm, such as Fast Fourier Transform (FFT), Mel Frequency Cepstral Coefficient (MFCC), Filter Bank and Wavelet, there is a difference in that an operation of obtaining a plurality of m-dimensional feature values from features 831, 832, 833 ... of a plurality of beat is a result of analysis using artificial intelligence.

When generalizing this to time series data, when time series data is split into n units and the features of each unit are extracted, it may be described as inputting the feature of each unit to the n intermediate neural networks. A feature of each of the n units is input to each input layer of the n intermediate neural networks to obtain m-dimensional feature values for each of the n units in the output layer of each of the n intermediate neural networks.

Next, the m-dimensional feature values for each of the n beats output from the n intermediate neural networks are input to the final neural network 850 in the order of the time axis of each beat. The final neural network may be the RNN. In this case, in order to reflect the time-varying features of consecutive beats, the m-dimensional feature value for the previous beat of the beat to be discriminated, the m-dimensional feature value for the beat to be discriminate and the m-dimensional feature value for the next beat of the beat to be discriminated may be sequentially input to the input layer of the final neural network.

Data input to the final neural network is output to c nodes in the output layer via the hidden layer, and when the final neural network is a classification model, the value output to the c nodes may be a value for classifying the entire electrocardiogram signal into c classifications. If the final neural network is a prediction model, the value output to the c nodes may be a value for predicting a result within a specific range from the entire electrocardiogram signal. The value output from the final neural network is referred to as the 'final output data.'

Since the final neural network receives the m-dimensional feature values for each beat sequentially, it is preferable that the nodes of the input layer of the final neural network are m. Of course, it is also possible to have m or more nodes in the input layer of the final neural network so that other values or parameters may be input in addition to the m-dimensional feature values for each beat.

As is commonly used in the technical field, to which the inventive concept belongs, the term 'classification model' herein refers to an artificial intelligence model for the purpose of finding out which group the input data belongs to, and the term 'prediction model' refers to an artificial intelligence model, in which the result value can be any value within the range of the learning data.

When generalizing this to time series data, when time series data is split into n units, n-dimensional feature values for each of n units are obtained using n intermediate neural networks. The final neural network receives m-dimensional feature values for each of the n units from the n intermediate neural networks in the order of the time axis of each unit, and performs tasks, such as classification and prediction of the entire time series data.

In order to reflect the time-varying features of consecutive units, the m-dimensional feature values of the unit to be discriminated and the m-dimensional feature values of the unit immediately adjacent to the unit to be discriminated may be sequentially input to the input layer of the final neural network. In the case of an electrocardiogram signal, three of the previous beat of the beat to be discriminated, the beat to be discriminated and the next beat of the beat to be discriminated should be sequentially input, but in the case of general time series data, only two of the previous unit of the unit to be discriminated and the unit to be discriminated or only two of the next unit of the unit to be discriminated and the unit to be discriminated may be input. Of course, it is also possible to input all three of the previous unit of the unit to be discriminated, the unit to be discriminated and the next unit of the unit to be discriminated.

Since the final neural network sequentially receives the m-dimensional feature values for each unit, it is preferable that the nodes of the input layer of the final neural network are m. Of course, it is also possible to have m or more nodes in the input layer of the final neural network so that other values or parameters may be input in addition to the m-dimensional feature values for each unit.

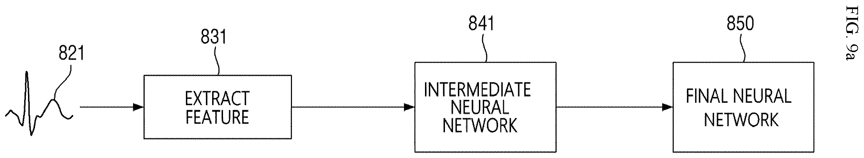

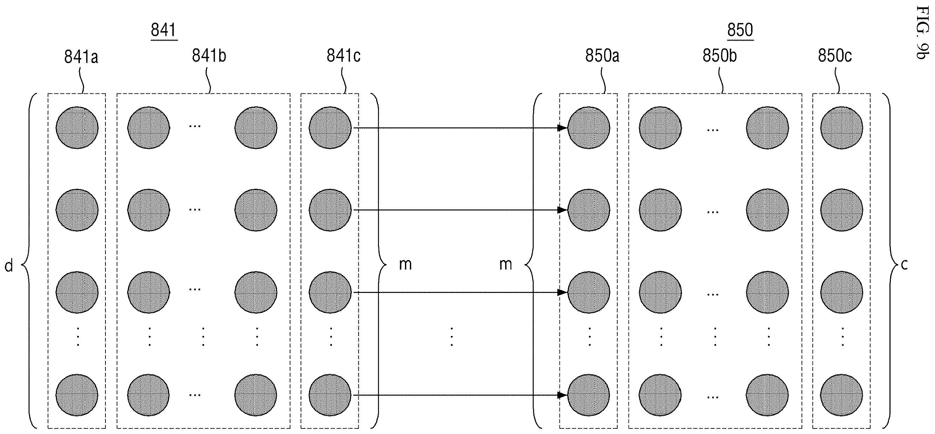

FIGS. 9a and 9b are diagrams illustrating node structures of an intermediate neural network and a final neural network used in a time series data analysis method using artificial intelligence according to an embodiment of the inventive concept.

For the convenience of description, FIGS. 9a and 9b illustrate that the feature 831 of the first beat 821 obtained by splitting the electrocardiogram signal 810 is input to the first intermediate neural network 841, and the intermediate output data of the first intermediate neural network 841 is input to the final neural network 850.

Assuming that a feature extracted from each beat using Fast Fourier Transform (FFT), Mel Frequency Cepstral Coefficient (MFCC), Filter Bank, Wavelet, etc. is a d-dimensional value, it is preferable that the number of nodes of the input layer of each intermediate neural network 841, 842, 843 is also d. Of course, it is also possible to have d or more nodes in the input layer of the intermediate neural network so that other values or parameters may be input in addition to the d-dimensional feature extracted from each beat.

When the d-dimensional feature 831 is extracted from the first beat 821, the d-dimensional feature 831 is input to d nodes in the input layer 841a of the first intermediate neural network 841. The input data is analyzed via the hidden layer 841b of the first intermediate neural network 841 and the analysis result is output at m nodes in the output layer 841c of the first intermediate neural network 841.

The intermediate output data of the first intermediate neural network 841 output from the m nodes in the output layer 841c of the first intermediate neural network 841 is input to the m nodes in the input layer 850a of the final neural network 850. The final neural network 850 not only receives the intermediate output data of the first intermediate neural network 841, but also receives intermediate output data from other intermediate neural networks 842, 843, and outputs the final output data after analyzing the time-varying features of consecutive beats via the hidden layer 850b. The final output data is output at c nodes in the output layer 850c of the final neural network 850.

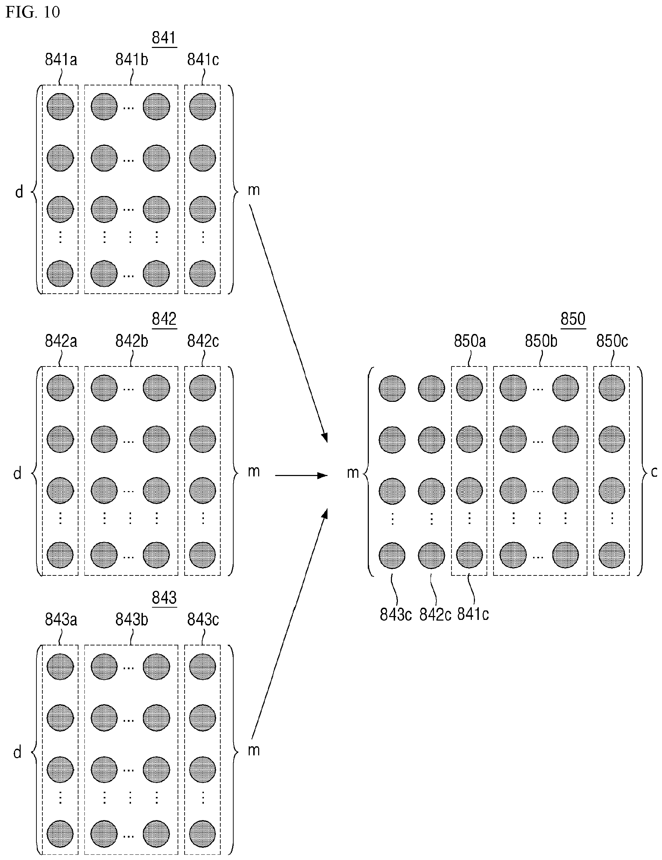

FIG. 10 is a diagram for describing a process of inputting intermediate output data output from a plurality of intermediate neural networks to a final neural network according to an embodiment of the inventive concept.

When the beat to be discriminated in the electrocardiogram signal is the second beat 822, the m-dimensional intermediate output data of the previous beat 821 of the beat to be discriminated, the beat 822 to be discriminated and the next beat 832 of the beat to be discriminated should be input sequentially.

That is, the m-dimensional intermediate output data 841c obtained by inputting the d-dimensional feature 831 extracted from the previous beat 821 of the beat to be discriminated to the first intermediate neural network 841, the m-dimensional intermediate output data 842c obtained by inputting the d-dimensional feature 832 extracted from the beat to be discriminated 822 to the second intermediate neural network 842 and,

the m-dimensional intermediate output data 843c obtained by inputting the d-dimensional feature 833 extracted from the next beat 823 of the beat to be discriminated to the third intermediate neural network 843 are sequentially input to the final neural network..

The above is summarized as follows.

Deep learning may be used to discriminate an arrhythmia by analyzing an electrocardiogram signal. The neural network model includes a CNN model and an RNN model. However, the CNN model is inadequate for time-series data analysis with time-varying features and is less accurate. Since the CNN method is based on the location information of the feature, there is a problem that the discriminating power is greatly reduced even for small deformation or noise. (Sabour, S., Frosst, N. Hinton, G. E. Dynamic routing between capsules.In Advances in Neural Information Processing Systems, pp. 3859-3869, 2017).

To overcome this problem, the RNN model can be used to analyze time series data with time-varying features. The biggest problem with the RNN model is that they are slow to learn and analyze. Although there is an improvement in accuracy because it can use more information and reflect time-varying features, it is more complex because more information needs to be remembered compared to the CNN. Further, since the association with consecutive units should be used as information, the consecutive units should be processed sequentially. Therefore, long inputs should be processed, which requires more time for learning and analysis.

According to an embodiment of the inventive concept, in order to solve the problem of low accuracy and the impossibility of variable length input processing of the CNN along with the problem of the slow learning and analysis speed of the RNN, Hierarchical RNN with variable length boundaries for parallelization model, which is composed of a plurality of intermediate neural networks and one final neural network, is proposed.

In the conventional RNN model, n consecutive beats were considered as one input. However, in the artificial intelligence model according to an embodiment of the inventive concept, the learning and analysis are divided into two stages of a short term RNN processed by a plurality of intermediate neural networks and a long term RNN processed by one final neural network. First, each of the n consecutive beats is used as an input of the intermediate neural network. As a result, an m-dimensional vector of one beat is obtained and inputted to the final neural network to analyze an electrocardiogram signal and discriminate a disease such as arrhythmia.

The plurality of intermediate neural networks process a plurality of beats, respectively, and the plurality of intermediate neural networks are the same model. Therefore, when processing a plurality of beats, parallel processing is possible because there is no dependency between each other. Therefore, compared to the conventional RNN method, in which all of the entire electrocardiogram signal should be inputted, the operation may be performed quickly in proportion to the number of computing devices that perform parallel processing or the number of processors that perform parallel processing. Of course, a structure, in which intermediate neural networks less than the number of a plurality of beats process a plurality of input beats, may be also possible depending on resource constraints. This structure processes each beat in parallel in a plurality of intermediate neural networks and processes shortened intermediate output data in the final neural network, thereby greatly reducing the number of nodes in the hidden layer of each neural network and increasing the available memory. This brings with it an improvement in the diagnostic speed in GPU environments that are constrained by video memory. Further, due to the shorter length of data to be processed in the final neural network, the complexity is reduced, which greatly improves the diagnostic speed.

The length of the input data when analyzing the electrocardiogram signal of FIG. 3 by using the time series data analysis method using artificial intelligence according to an embodiment of the inventive concept is calculated as follows.

The electrocardiogram signal of FIGS. 3a and 3b is divided into nine beats. Assuming that one beat is sampled at 250 Hz, the processing length when extracting features from nine beats is 250 × 9 = 2250.

When nine features extracted from nine beats are input to nine intermediate neural networks, the processing length is 1 × 9 = 9.

When the m-dimensional intermediate output data output from the nine intermediate neural networks is input to the final neural network, m-dimensional intermediate output data for the previous bit, the discrimination target beat and the next beat are sequentially input. As described above, data is input to the final neural network in the following order.
(1) The m-dimensional feature value for beat A, the m-dimensional feature values for beat B and the m-dimensional feature value for beat C.
(2) The m-dimensional feature value for beat B, the m-dimensional feature values for beat C and the m-dimensional feature value for beat D.
(3) The m-dimensional feature value for beat C, the m-dimensional feature values for beat D and the m-dimensional feature value for beat E
(4) The m-dimensional feature value for beat D, the m-dimensional feature values for beat E and the m-dimensional feature value for beat F.
(5) The m-dimensional feature value for beat E, the m-dimensional feature values for beat F and the m-dimensional feature value for beat G.
(6) The m-dimensional feature value for beat F, the m-dimensional feature values for beat G and the m-dimensional feature value for beat H.
(7) The m-dimensional feature value for beat G, the m-dimensional feature values for beat H and the m-dimensional feature value for beat I.

That is, since the three-length input is made seven times, the length to be processed is 3 × 7 = 21 when the m-dimensional intermediate output data outputted from the nine intermediate neural networks are input to the final neural network.

Adding up all the processing lengths is 2250 + 9 + 21 = 2280. It can be seen that the processing length was reduced by one third as compared to the processing length of the conventional method illustrated in FIGS. 3a and 3b being 5250.

As described above, according to the time series data analysis method using artificial intelligence according to an embodiment of the inventive concept, the accuracy of the analysis is higher than that of the conventional CNN-based electrocardiogram analysis method, and the length of data to be processed is shortened compared to the conventional RNN-based electrocardiogram analysis method, thereby increasing the speed of analysis and ensuring real-time electrocardiogram analysis. Through this, the electrocardiogram signal can be accurately analyzed in real time and the subject is notified before the heart disease occurs, so that it is possible to take action in advance with medication or hospital treatment. Furthermore, since agile initial response to fatal heart disease is possible, it can be used for urgent lifesaving services.

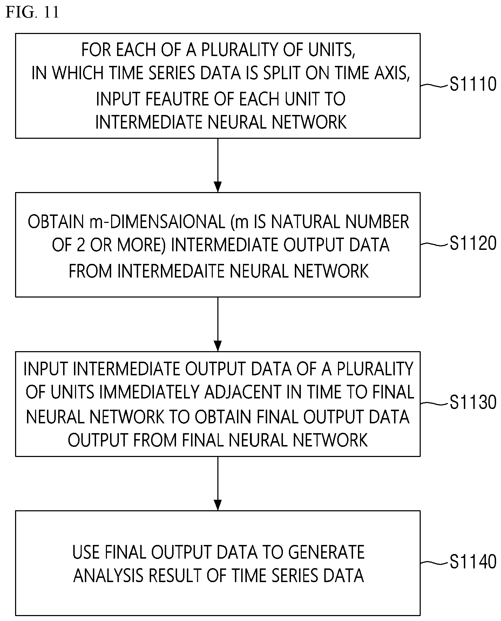

FIG. 11 is a flowchart illustrating a time series data analysis method using artificial intelligence according to an embodiment of the inventive concept.

As shown in FIG. 11, the time series data analysis method using artificial intelligence according to an embodiment of the inventive concept may comprise, for each of a plurality of units, in which time series data is split on a time axis, the step of inputting the feature of each unit to an intermediate neural network (S1110), the step of obtaining the m-dimensional (m is a natural number of 2 or more) intermediate output data from the intermediate neural network (S1120), the step of inputting intermediate output data of a plurality of units immediately adjacent in time to the final neural network to obtain final output data output from the final neural network (S1130) and the step of generating the analysis result of time series data using the final output data (S1140).

Since each step has been described in detail above, the duplicate description is omitted.

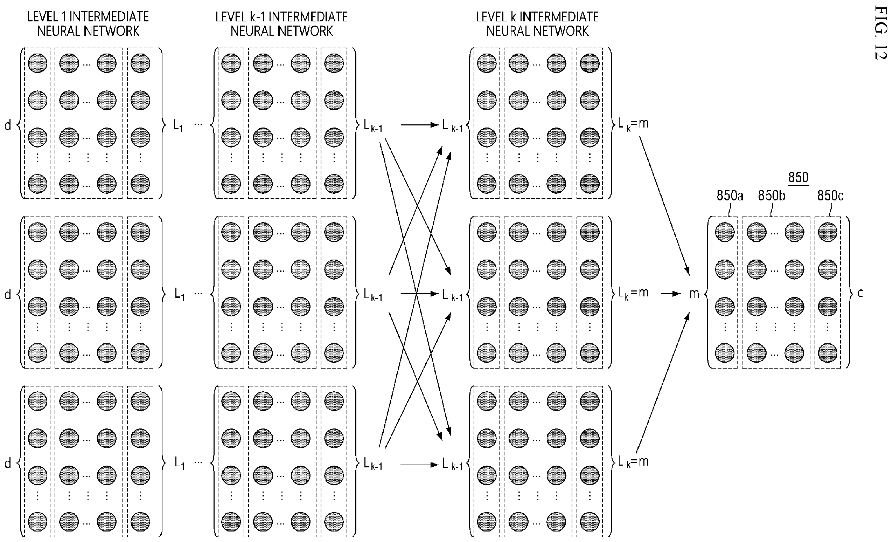

FIG. 12 is a diagram illustrating a case, where an intermediate neural network is implemented with a plurality of levels in a neural network structure according to an embodiment of the inventive concept.

As shown in FIG. 12, in the time series data analysis method using artificial intelligence according to an embodiment of the inventive concept, the intermediate neural network can be implemented with a plurality of levels.

The intermediate neural network of each level outputs the feature value of the input data and the feature value output from the intermediate neural network of the previous level is input to the intermediate neural network of the next level.

When the intermediate neural network is composed of k levels, the feature of each of the plurality of units is input to the level 1 intermediate neural network, and the feature value output from the intermediate neural network of each level is input to the intermediate neural network of the next level. The feature value output from the intermediate neural network of level k-1 is input to the intermediate neural network of level k and the m-dimensional feature value output from the intermediate neural network of level k is input to the final neural network.

In FIG. 12, the intermediate neural network having a plurality of levels is designed in such a manner that a plurality of feature values output from the plurality of intermediate neural networks of the previous level are input to one intermediate neural network of the next level, which illustrates that data of each unit (first unit) is processed in the level 1 intermediate neural network, data of the second unit grouping a plurality of units adjacent in time is processed in the level 2 intermediate neural network and data of the third unit grouping a plurality of units of the second unit adjacent in time is processed in the level 3 intermediate neural network. In this case, the m-dimensional feature value output from the intermediate neural network of level k will be the m-dimensional feature value for the data of the kth unit. In this case, the number of intermediate neural networks of each level may vary according to the grouping criterion.

Of course, the intermediate neural network having a plurality of levels may be designed in such a manner that one feature value output from one intermediate neural network of the previous level is input 1:1 to one intermediate neural network of the next level. In this case, the intermediate neural networks of each level can be implemented with the same number. For example, when the number of level 1 intermediate neural networks is n, the number of level 2 intermediate neural networks may be implemented as n, the number of level 3 intermediate neural networks may be implemented as n and the number of level k intermediate neural networks also may be implemented as n.

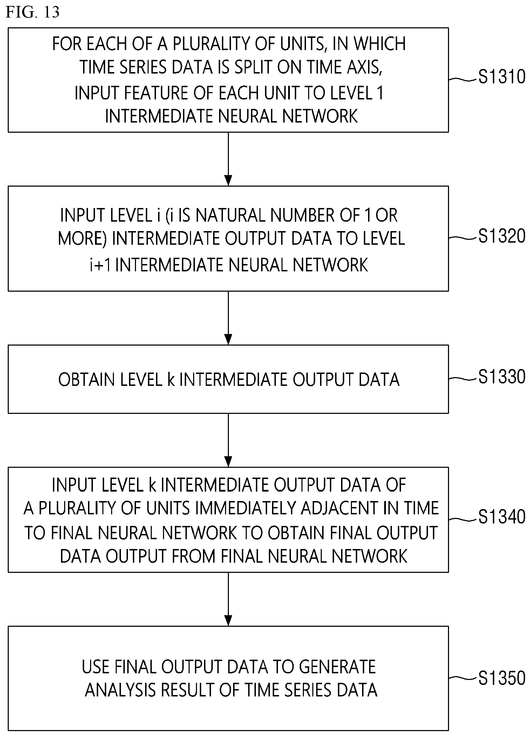

FIG. 13 is a flowchart illustrating a time series data analysis method when an intermediate neural network is implemented with a plurality of levels in a neural network structure according to an embodiment of the inventive concept.

In the time series data analysis method using artificial intelligence according to an embodiment of the inventive concept, when the intermediate neural network has a plurality of levels, the time series data analysis method may comprises, for each of a plurality of units, in which time series data is split on a time axis, the step of inputting a feature of each unit to the level 1 intermediate neural network (S1310), the step of inputting intermediate output data output from the level i (i is a natural number of 1 or more) neural network to the level i+1 intermediate neural network (S1320), the step of obtaining intermediate output data from the level k intermediate neural network (S1330) and the step of sequentially inputting level k intermediate output data to the final neural network to obtain final output data from the final neural network (S1340).

If the intermediate neural network having a plurality of levels is designed in such a manner that a plurality of feature values output from the plurality of intermediate neural networks of the previous level are input to one intermediate neural network of the next level, the step of sequentially inputting level k intermediate output data to the final neural network may include sequentially inputting level k intermediate output data for the data of the kth unit immediately adjacent in time to the final neural network.

Meanwhile, if the intermediate neural network having a plurality of levels is designed in such a manner that one feature value output from one intermediate neural network of the previous level is input 1: 1 to one intermediate neural network of the next level, the step of sequentially inputting the level k intermediate output data for immediately adjacent units in time to the final neural network is the same as the case, where the number of levels of the intermediate neural network is one described with reference to FIGS. 9 and 10, and thus the duplicated description is omitted..

In the above, the time series data learning method using artificial intelligence according to an embodiment of the inventive concept has been described.

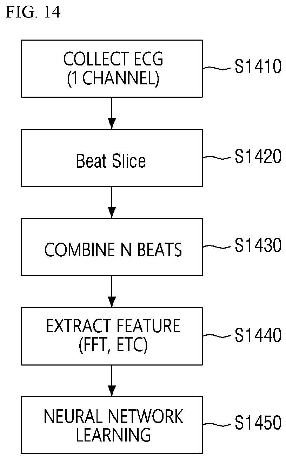

Hereinafter, a time series data learning method using artificial intelligence according to an embodiment of the inventive concept will be described with reference to FIGS. 14 to 17. In detail, a process of learning an electrocardiogram signal using a plurality of neural networks according to an embodiment of the inventive concept will be described to explain a time series data learning method using artificial intelligence according to an embodiment of the inventive concept.

As is well known, the learning process of neural network using learning data is basically similar to the analysis process of input data using neural network in many parts. However, in the learning process of neural network, there is a difference in that the step of calculating the error by comparing the output value and the actual value and changing the weight of the neural network by back propagating the error is added.

Hereinafter, a time series data learning method according to an embodiment of the inventive concept will be described based on differences from the time series data analysis method according to an embodiment of the inventive concept.

FIG. 14 is a flowchart illustrating an electrocardiogram learning method using artificial intelligence according to an embodiment of the inventive concept.

As shown in FIG. 14, an electrocardiogram learning method using artificial intelligence according to an embodiment of the inventive concept may comprise an electrocardiogram signal collecting step (S1410), an electrocardiogram signal splitting step (S1420), a beat combining step (S1430), a feature extracting step (S1440) and a neural network learning step (S1450).

The electrocardiogram signal collecting step (S1410) is a step, in which the server 200 collects the electrocardiogram signal received from the electrocardiogram measuring device 100 or the electrocardiogram signal directly input by the user. In general, the entire electrocardiogram signal measured for a long time is not labeled with the discriminant result, such as arrhythmias, but is labeled with the discriminant result in the unit of beat, such as arrhythmias. Well-known data includes the MIT-BIH Arrhythmia Database.

The electrocardiogram signal splitting step (S1421) is a step of splitting the electrocardiogram signal by the unit of beat.

The beat combining step (S1430) is a step of combining a of previous beats and b of next beats with the labeled beat.

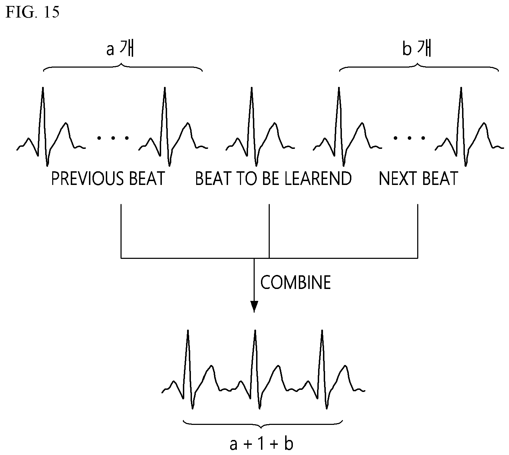

FIG. 15 is a diagram illustrating learning data used in an electrocardiogram learning method using artificial intelligence according to an embodiment of the inventive concept.

As shown in FIG. 15, an electrocardiogram learning method using artificial intelligence according to an embodiment of the inventive concept combines a of previous beats and b of next beats with the labeled beat and uses them as learning data.

A time series data analysis method using artificial intelligence according to an embodiment of the inventive concept does not analyze a single beat, but analyzes a time-varying features together generated by concatenating a plurality of adjacent beats in time, such as the occurrence interval with a previous beat or a next beat, the height difference with a previous beat or a next beat. Therefore, in learning as well, the labeled beat is not learned alone, but the previous beat and the next beat should be combined with the labeled beat and learned.

The number of previous beats a and the number of next beats b to be combined with the labeled beat can be arbitrarily determined. The number of previous beats a and the number of next beats b, which are determined at this time, should be equally used as an input for analysis.

The feature extracting step (S 1440) is a step of extracting a feature of each beat. Feature extraction can use a variety of methods widely used in signal processing, such as Fast Fourier Transform (FFT), Mel Frequency Cepstral Coefficient (MFCC), Filter Bank and Wavelet. The feature extraction algorithm used for learning and the feature extraction algorithm used for analysis are preferably same.

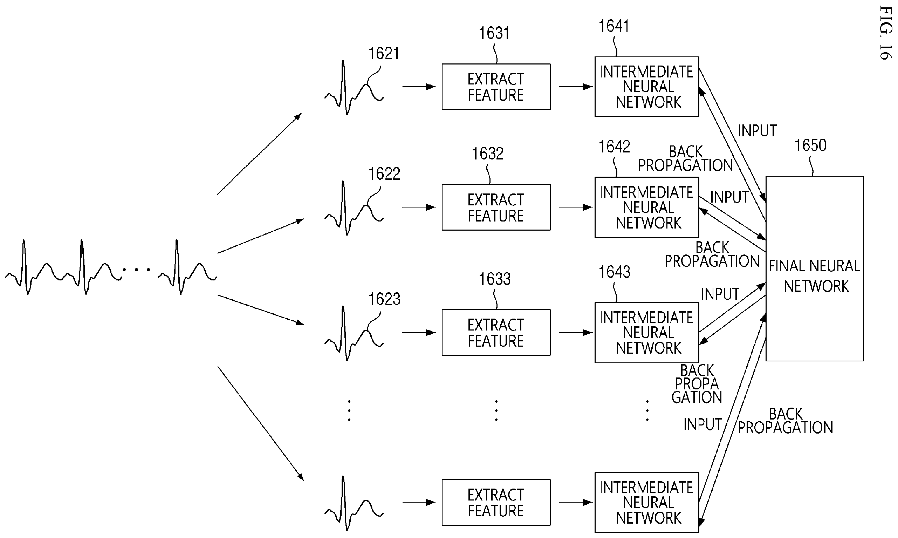

The neural network learning step (S1450) is a step of learning the neural network by using well-known techniques, such as back propagation and gradient descent. Hereinafter, the neural network learning step (S1450) will be described in detail with reference to FIG. 16.

FIG. 16 is a diagram illustrating a time series data learning method using artificial intelligence according to an embodiment of the inventive concept.

A process of learning time series data, for example an electrocardiogram signal, using artificial intelligence according to an embodiment of the inventive concept will be described with reference to FIG. 16.

When using a database labeled by the unit of beat, such as the MIT-BIH Arrhythmia Database, the learning data 1610 should be an electrocardiogram signal, in which one or more previous beats and one or more next beats are combined with a labeled beat through the beat combining step (S1430). If a database, in which each electrocardiogram signal is labeled with the result of analyzing the entire electrocardiogram signal instead of the unit of beat, is used, the labeled electrocardiogram signal may be used as the learning data 1610 without including the beat combining step (S1430).

Generalizing this to time series data, when using a database labeled by the unit of unit, the learning data should be time series data combining one or more units with a labeled unit. If a database, in which each electrocardiogram data is labeled with the result of analyzing the entire electrocardiogram signal instead of the unit of beat, is used, the labeled time series data may be used as learning data.

When the learning data 1610 is input, the learning data is split into a plurality of beats 1621, 1622, 1623 ... according to a predetermined criterion. The electrocardiogram signal splitting method used in learning and the electrocardiogram signal splitting method used in analysis are preferably the same.

When generalizing this to time series data, when learning data is input, it can be described as splitting learning data into a plurality of units according to a predetermined criterion. The splitting method of time series data used in learning and the splitting method of time series data used in analysis are preferably the same.

Next, for each of the plurality of beats 1621, 1622, 1623..., an operation of extracting the features 1631, 1632, 1633... of each beat is performed. The feature extraction algorithm of the electrocardiogram signal used in learning and the feature extraction algorithm of the electrocardiogram signal used in analysis are preferably the same.

When generalizing this to time series data, when time series data is split into a plurality of units, it may be described as extracting a feature of each unit for each of the plurality of units. The feature extraction algorithm of the time series data used in learning and the feature extraction algorithm of the time series data used in analysis are preferably the same.

[Next, an operation of inputting the features 1631, 1632, 1633... of each beat into the intermediate neural network 1641, 1642, 1643 is performed. If the learning data 1610 is the combination of n beats, then all n features would have been extracted, in which case the intermediate neural networks are preferably n.

When generalizing this to time series data, when time series data is split into n units and the features of each unit are extracted, it may be described as inputting the features of each unit to the n intermediate neural networks.

Next, the m-dimensional feature values for each of the n beats output from the n intermediate neural networks are input to the final neural network 1650 in the order of the time axis of each beat to obtain final output data for the learning data..

When generalizing this to time series data, when time series data is split into n units, the m-dimensional feature values for each of n units are obtained using n intermediate neural networks, and the final neural network obtains classification values, prediction values, and the like for the learning data by receiving m-dimensional feature values for each of the n units from the n intermediate neural networks in the order of the time axis of each unit.

When the final output data for the learning data is obtained from the final neural network 1650, the final output data for the learning data is compared with a label attached to the learning data. At this time, the final output data for the learning data is an output value and the label attached to the learning data is an actual value. The error between the output value and the actual value is calculated using a loss function or the like and then back propagated.

The weight of the final neural network 1650 and the weights of the intermediate neural networks 1641, 1642, 1643... are adjusted through the back propagation. The back propagation and the weight adjustment method of a neural network are described in detail in the literature, such as https://en.wikipedia.org/wiki/Backpropagation, so the detailed description is omitted.

At this time, it should be noted as of the setting of the learning rate of the final neural network 1650 and intermediate neural networks 1641, 1642, 1643... The term 'learning rate' refers to the size of the step to find out the lowest value in the gradient descent algorithm for weight adjustment of the neural network.

Intermediate neural networks cannot learn in parallel. This is because there is only one correct answer for a plurality of input beats. Because of this, back propagation should be performed for a plurality of input beats for one correct answer. If the number of beats is n and the number of intermediate neural networks is n, the n intermediate neural networks should share weights with each other, and the learning rate of the intermediate neural network in back propagation should be set as 1/n times of the learning rate of the final neural network.

Meanwhile, even if the intermediate neural network is implemented with a plurality of levels, the learning method of the neural network is the same. After analyzing the learning data in the same manner as the analysis method of the neural network when the intermediate neural network is implemented with a plurality of levels (see FIGS. 12 and 13), when the final output data for the learning data is obtained from the final neural network, the final output data (output value) for the learning data is compared with the label (actual value) attached to the learning data. The error between the output value and the actual value is calculated using a loss function or the like and then back propagated to adjust the weight of the final neural network and the weights of the intermediate neural networks of the plurality of levels.

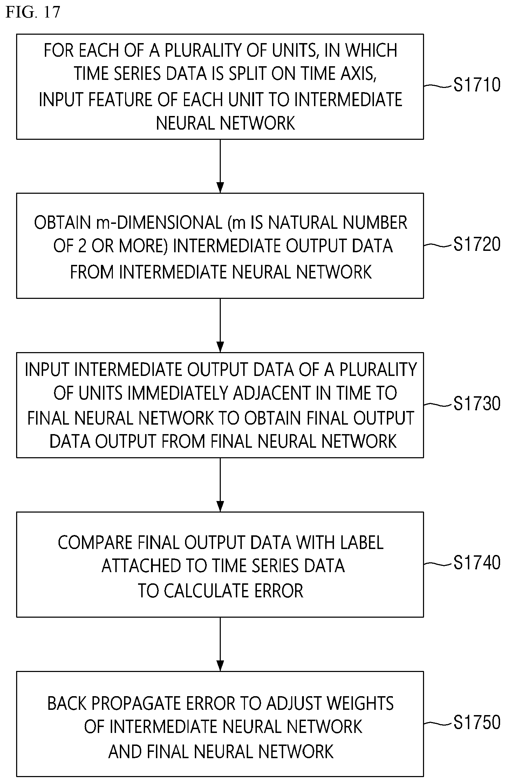

FIG. 17 is a flowchart illustrating a time series data learning method using artificial intelligence according to an embodiment of the inventive concept.

As shown in FIG. 17, the time series data learning method using artificial intelligence according to an embodiment of the inventive concept may comprise, for each of a plurality of units, in which time series data is split on a time axis, the step of inputting a feature of each unit to the intermediate neural network (S1710), the step of obtaining m-dimensional (m is a natural number of 2 or more) intermediate output data from the intermediate neural network (S1720), inputting intermediate output data of a plurality of units immediately adjacent in time to the final neural network to obtain the final output data output from the final neural network (S1730), the step of comparing the final output data with a label attached to time series data to calculate an error (S1740) and the step of back propagating the error to adjust the weights of the intermediate neural network and the final neural network (S1740).

Since each step has been described in detail above, the duplicated description is omitted.

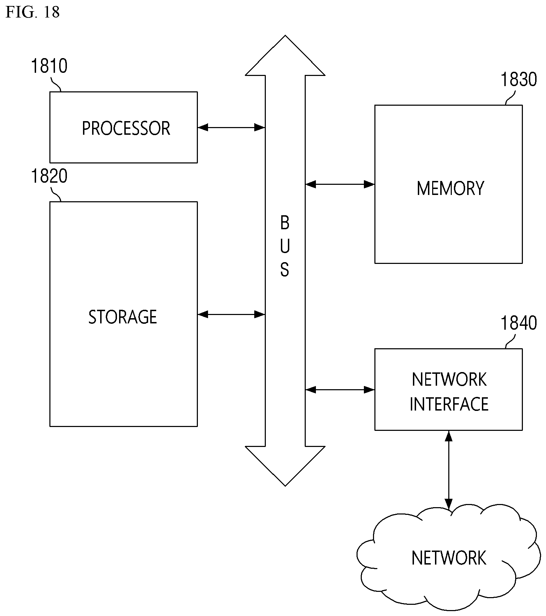

FIG. 18 is a hardware configuration diagram of an exemplary computing device that may implement a time series data analysis method or time series data learning method using artificial intelligence according to embodiments of the inventive concept.

As shown in FIG. 18, an exemplary computing device that may implement a time series data analysis method or a time series data learning method using artificial intelligence according to embodiments of the inventive concept may include one or more processors 1810, a storage 1820, a memory 1830 that loads a computer program performed by the processor 1810, a network interface 1840 and a bus. FIG. 18 shows only components relevant to the embodiments of the inventive concept. Therefore, it will be appreciated by those skilled in the art that the inventive concept may further include other general purpose components in addition to the components illustrated in FIG. 18.

The processor 1810 controls the overall operation of each component of the computing device that may implement the time series data analysis method or the time series data learning method using artificial intelligence according to embodiments of the inventive concept. The processor 1810 may be configured to include a central processing unit (CPU), a micro processor unit (MPU), a micro controller unit (MCU), a graphics processing unit (GPU), or any type of processor well known in the art. Further, the processor 1810 may perform an operation on at least one application or program for executing a method according to embodiments of the inventive concept. A computing device that may implement a time series data analysis method or a time series data learning method using artificial intelligence according to embodiments of the inventive concept may include one or more processors.

The storage 1820 can non-temporarily store one or more computer programs. The storage 1820 may be configured to include a non-volatile memory such as a read only memory (ROM), erasable programmable ROM (EPROM), electrically erasable programmable ROM (EEPROM), flash memory and the like, a hard disk, a removable disk or any computer readable recording medium well-known in the technical field, to which the inventive concept belongs. The storage 1820 stores a computer program for performing the time series data analysis method or the time series data learning method using artificial intelligence according to the embodiments of the inventive concept.

The memory 1830 stores various data, commands, and/or information. The memory 1830 may load one or more computer programs from the storage 1820 to execute the time series data analysis method or the time series data learning method using artificial intelligence according to the embodiments of the inventive concept.

The bus provides a communication function between components of a computing device that may implement the time series data analysis method or the time series data learning method using artificial intelligence according to embodiments of the inventive concept. The bus may be implemented as various types of buses such as an address bus, a data bus and a control bus.

The network interface 1840 supports wired/wireless communication of a computing device that may implement the time series data analysis method or the time series data learning method using artificial intelligence according to the embodiments of the inventive concept. Further, the network interface 1840 may support various communication methods other than Internet communication. To this end, the network interface 1840 may be configured to include a communication module well known in the art.

A computer program for performing the time series data analysis method or the time series data learning method using artificial intelligence according to the embodiments of the inventive concept may include instructions, which is loaded in the memory 1830 and causes the processor 1810 to perform the time series data analysis method or the time series data learning method according to the embodiments of the inventive concept.

Hereinafter, a time series data analysis apparatus using artificial intelligence according to embodiments of the inventive concept will be described.

The time series data analysis apparatus according to an embodiment of the inventive concept includes a network interface 1840 for receiving time series data, one or more processors 1810, a memory 1830 for loading a computer program performed by the processor 1841 and a storage 1820 for storing a computer program, wherein the computer program may comprise, for each of a plurality of units, in which time series data is split on a time axis, an instruction for inputting a feature of each of the units to an intermediate neural network and obtaining m-dimensional (m is a natural number of 2 or more) intermediate output data, an instruction for inputting intermediate output data of a plurality of units immediately adjacent in time to a final neural network and obtaining final output data output from the final neural network and an instruction for using the final output data to generate an analysis result of time series data.

Since each instruction of the computer program stored in the storage 1820 of the time series data analysis apparatus according to an embodiment of the inventive concept performs the time series data analysis method using artificial intelligence described above, the duplicated description is omitted.

Hereinafter, a time series data learning apparatus using artificial intelligence according to embodiments of the inventive concept will be described.

The time series data learning apparatus according to an embodiment of the inventive concept may include a network interface 1840 for receiving time series data, one or more processors 1810, a memory 1830 for loading a computer program performed by the processor 1841 and a storage 1820 for storing a computer program, wherein the computer may comprise, for each of a plurality of units, in which time series data is split on a time axis, an instruction for inputting a feature of each of the units to an intermediate neural network and obtaining m-dimensional (m is a natural number of 2 or more) intermediate output data, an instruction for inputting intermediate output data of a plurality of units immediately adjacent in time to a final neural network and obtaining final output data output from the final neural network and an instruction for comparing the final output data with a label attached to the time series data.

Since each instruction of the computer program stored in the storage 1820 of the time series data learning apparatus according to an embodiment of the inventive concept performs the time series data learning method using artificial intelligence described above, the duplicated description is omitted.

The concepts of the invention described with reference to the drawings can be embodied as computer-readable code on a computer-readable medium. The computer-readable medium may be a removable recording medium (a CD, a DVD, a Blu-ray disc, a USB storage device, or a removable hard disc) or a fixed recording medium (a ROM, a RAM, or a computer-embedded hard disc). The computer program recorded on the computer-readable recording medium may be transmitted to another computing apparatus via a network such as the Internet and installed in the computing apparatus. Hence, the computer program can be used in the computing apparatus.

Although operations are shown in a specific order in the drawings, it should not be understood that desired results can be obtained when the operations must be performed in the specific order or sequential order or when all of the operations must be performed. In certain situations, multitasking and parallel processing may be advantageous. According to the above-described embodiments, it should not be understood that the separation of various configurations is necessarily required, and it should be understood that the described program components and systems may generally be integrated together into a single software product or be packaged into multiple software products.

While embodiments of the invention concept have been described with reference to the attached drawings, it will be understood by those of ordinary skill in the art that various changes in form and detail may be made therein without changing the spirit and the features of the present invention. The exemplary embodiments should be considered in a descriptive sense only and not for purposes of limitation.

## Claims

1. A time series data analysis method performed by a computing device, the method comprising:
inputting, for each of a plurality of units, in which the time series data is split on a time axis, a feature of each of the units to an intermediate neural network;
obtaining m-dimensional intermediate output data (m is a natural number of 2 or more) from the intermediate neural network;
inputting the intermediate output data of a plurality of units immediately adjacent in time to a final neural network to obtain final output data output from the final neural network; and
generating an analysis result of the time series data using the final output data.

2. The time series data analysis method of claim 1,
wherein the intermediate neural network and the final neural network are RNN (Recurrent Neural Network).

3. The time series data analysis method of claim 1,
wherein nodes of an input layer of the final neural network are m.

4. The time series data analysis method of claim 1,
wherein obtaining the intermediate output data comprises,
inputting, by a first computing device, a feature of a first unit included in the plurality of units to the intermediate neural network implemented in the first computing device and obtaining m-dimensional intermediate output data from the intermediate neural network; and
inputting, by a second computing device, a feature of a second unit included in the plurality of units to an intermediate neural network implemented in the second computing device and obtaining m-dimensional intermediate output data from the intermediate neural network.

5. The time series data analysis method of claim 4,
wherein obtaining the final output data comprises,
receiving, by a third computing device, intermediate output data of the first unit from the first computing device, and intermediate output data of the second unit from the second computing device, and sequentially inputting intermediate output data of the first unit and intermediate output data of the second unit to the final neural network, wherein the first unit and the second unit are immediately adjacent units in time.

6. The time series data analysis method of claim 1,
wherein the intermediate neural network comprises k levels (k is a natural number of 2 or more),
wherein inputting to the intermediate neural network comprises,
inputting a feature of each of the plurality of units to a level 1 intermediate neural network;
inputting level i (i is a natural number of 1 or more) output data obtained from a level i intermediate neural network to a level i+1 intermediate neural network; and
obtaining level i + 1 output data from a level i + 1 intermediate neural network, wherein the intermediate output data is data output from a level k intermediate neural network.

7. The time series data analysis method of claim 1,
wherein the time series data is electrocardiogram data.

8. The time series data analysis method of claim 7,
wherein the analysis result is an electrocardiogram diagnosis result.

9. The time series data analysis method of claim 7,
wherein the n units are beat units that are split using R-peak.

10. The time series data analysis method of claim 9,
wherein the split n beat units are obtained by splitting the time series data and comparing similarities between split beats to replace a beat having a similarity to a preceding specific beat equal to or more than a predetermined value with the feature unit.

11. A time series data learning method performed by a computing device, the method comprising:
inputting, for each of a plurality of units, in which the time series data is split on a time axis, a feature of each of the units to an intermediate neural network;
obtaining m-dimensional (m is a natural number of 2 or more) intermediate output data from the intermediate neural network;
inputting the intermediate output data of a plurality of units immediately adjacent in time to a final neural network to obtain final output data output from the final neural network; and
comparing the final output data with a label attached to the time series data.

12. The time series data learning method of claim 11,
wherein the intermediate neural network and the final neural network are RNN (Recurrent Neural Network).

13. The time series data learning method of claim 11,
wherein nodes of an input layer of the final neural network are m.

14. The time series data learning method of claim 11,
wherein the comparing step comprises,
comparing the final output data with a label attached to the time series data to calculate an error,
the method further comprises,
back propagating the error to adjust a weight of the intermediate neural network and a weight of the final neural network after the comparing step.

15. The time series data learning method of claim 14,
wherein adjusting the weight comprises,
adjusting a weight of the intermediate neural network by making a learning rate of the intermediate neural network 1/n times a learning rate of the final neural network.

16. The time series data learning method of claim 11,
wherein the intermediate neural network comprises k levels (k is a natural number of 2 or more),
wherein inputting to the intermediate neural network comprises,
inputting a feature of each of the plurality of units to a level 1 intermediate neural network;
inputting level i (i is a natural number of 1 or more) output data obtained from a level i intermediate neural network to a level i+1 intermediate neural network; and
obtaining level i+1 output data from a level i+1 intermediate neural network, wherein the intermediate output data is data output from a level k intermediate neural network.

17. The time series data learning method of claim 11,
wherein the time series data is electrocardiogram data.

18. The time series data learning method of claim 17,
wherein the n units are beat units that are split using a R-peak.

19. A time series data analysis apparatus comprising:
a network interface for receiving time series data;
one or more processors;
a memory for loading a computer program performed by the processor; and
a storage for storing the computer program,
wherein the computer program comprises,
an instruction for inputting, for each of a plurality of units, in which the time series data is split on a time axis, a feature of each of the units to an intermediate neural network and obtaining m-dimensional (m is a natural number of 2 or more) intermediate output data from the intermediate neural network;
an instruction for inputting the intermediate output data of a plurality of units immediately adjacent in time to a final neural network and obtaining final output data output from the final neural network; and
an instruction for generating an analysis result of the time series data using the final output data.

20. A time series data learning apparatus comprising:
a network interface for receiving time series data;
one or more processors;
a memory for loading a computer program performed by the processor; and
a storage for storing the computer program,
wherein the computer program comprises,
an instruction for inputting, for each of a plurality of units, in which the time series data is split on a time axis, a feature of each of the units to an intermediate neural network and obtaining m-dimensional (m is a natural number of 2 or more) intermediate output data from the intermediate neural network;
an instruction for inputting the intermediate output data of a plurality of units immediately adjacent in time to a final neural network and obtaining final output data output from the final neural network; and
an instruction for comparing the final output data with a label attached to the time series data.
